# EUROPEAN PATENT APPLICATION

(11) **EP 1 120 106 A1**
(43) Date of publication of application: **01.08.2001**
(21) Application number: 00101251.7
(22) Date of filing: 25.01.2000
(51) Int. Cl.: A61K 7/16, A61K 7/18

(54) **Method for sealing dentinal tubules through the in-situ formation of low-solubility precipitates**

(71) Applicant: Neirinckx, Rudi Dominique, Dr., 68440 Dietwiller (FR)
(72) Inventor: Neirinckx, Rudi Dominique, Dr., 68440 Dietwiller (FR)

(57) **Abstract**

It was found that the combination of an alkaline earth or other cation , together with a suitable anion , when applied simultaneously -in vivo or in-vitro - slowly form microcrystalline deposits which block the tubuli of dentine and lower the pain caused by dentinal sensitivity.

The data presented consist of scanning electron microscopic images, which indicate the mechanism by which the pain may be lowered , and clinical results obtained in a pilot trial using strontium as the cation and sodium fluorophosphates as a source for the anions fluoride and phosphate , demonstrating the validity of the in-vitro findings.

## Description

The following application concerns the finding that the combination of an alkaline earth or other cation , in combination with a suitable anion , when applied simultaneously in vivo slowly form microcrystalline deposits which can block the tubuli of dentine and thus can lower the pain caused by dentinal sensitivity.

The data presented consist of scanning electron microscopic images, which indicate the mechanism by which the pain may be lowered , and clinical results obtained in a pilot trial using strontium as the cation and sodium fluorophosphates as a source for the anions fluoride and phosphate , demonstrating the validity of the in-vitro findings.

Several series of in-vitro data were collected:
1. Experiments using toothpaste formulations and mouthwash formulations to demonstrate the enhanced uptake of strontium on whole extracted teeth when using an in-situ mixture of strontium chloride (labeled with Sr-85) and sodium fluoride.
2. Images obtained with the scanning electron microscope of extracted teeth, treated with strontium chloride solutions or with in-situ mixtures of strontium chloride and sodium fluoride.
3. Studies of the precipitation rate of strontium salts, using strontium chloride and sodium fluoride or sodium fluorophosphates with or without saliva.
4. Study of the effect of pH of the Strontium solutions on the permeability of dentinal plates.

Clinically, one group of dentinal-sensitivity sufferers was treated with a double-phase mouthwash consisting of separate phases of strontium chloride and sodium fluoride while a parallel group was treated with a placebo. The remaining degree of sensitivity was tested by challenging the teeth with either a blast of cold air or by exerting physical pressure on the tooth. The degree of pain can only be measured subjectively and was noted on a visual-analog scale.

The results of this program of work demonstrated that:
1. The deposition of strontium on teeth is increased when applied in conjunction with fluoride.
2. Strontium salts are deposited on the tooth and in the dentinal channels using the two-phase formulation, not when using strontium chloride only.
3. Precipitates form slowly - as is desired - from strontium chloride and either sodium fluoride or sodium fluorophosphates in the presence of saliva. In the absence of saliva sodium fluorophosphates do not decompose and a precipitate is not formed.
4. The permeability of dentine is significantly increased when the acidity of the bathing solution is increased and the effect can be countered with the two-phase formulation.
5. The clinical efficacy of the product was demonstrated through a significant reduction in the pain levels experienced by the sufferers who used the two-phase formulation, when compared to the relief felt by the placebo users. This was valid for both challenging procedures and was consistent over time.
6. The healing effect of the formulation appears to be long-term, as demonstrated by the low levels of sensitivity remaining even 6 weeks after the end of treatment.

### RESULTS

### 1. SR-DEPOSITION ON TEETH

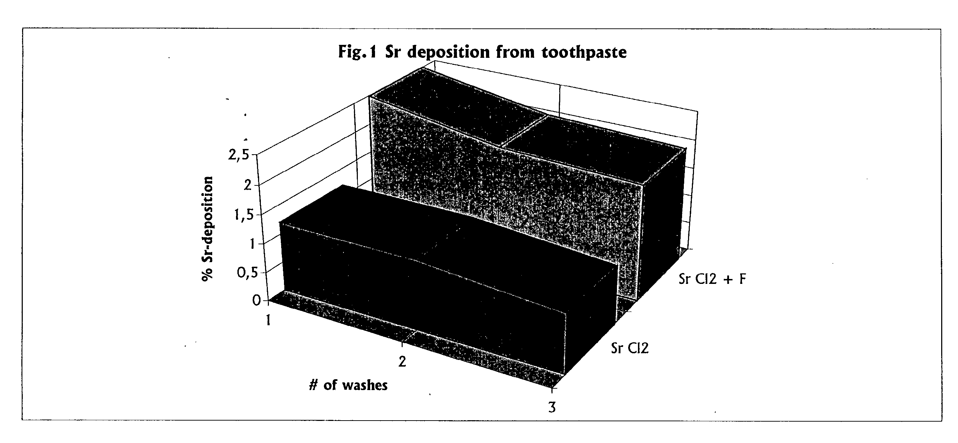

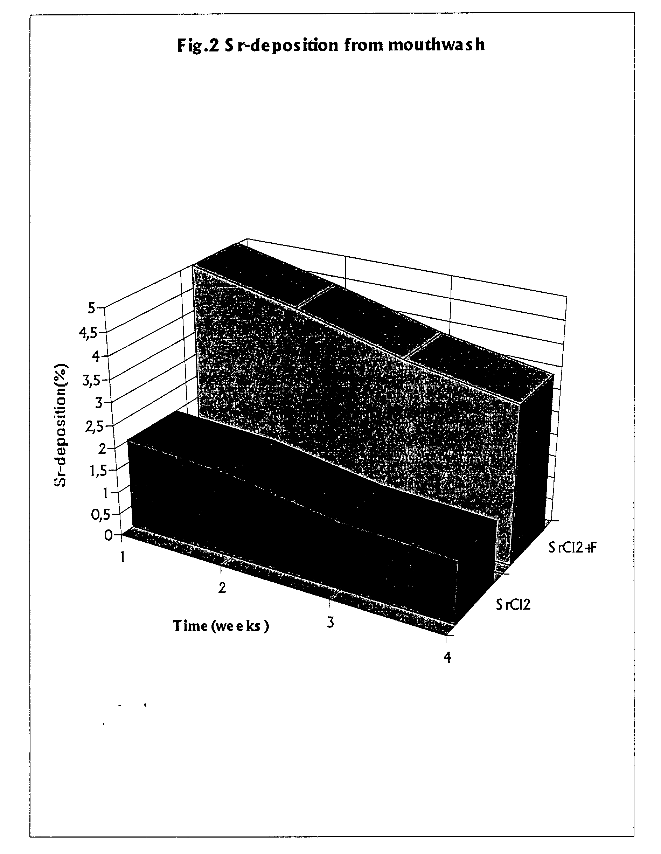

Figures 1 and 2 ( Addendum ) depict the deposition of the % of strontium onto the teeth from the novel formulations.

### 2. TOOTH DEPOSITION

Experimental mouthrinse formulations and a control were tested for their efficacy at depositing Strontium upon the tooth surfaces. The controls consisted of essentially the same ingredients as the tested actives, apart from their inability to release fluoride in the experimental conditions. Their formulation was a good mimick of "strontium-only" products such as Sensodyne.

Scanning electron microscopy identified the presence of crystals on dentin treated with the novel mouthwash Similar crystals were found in their suspensions when they were passed through Nucleopore filters designed to trap particulates in solutions. No other crystals were detected in solution or on dentin from the controls.

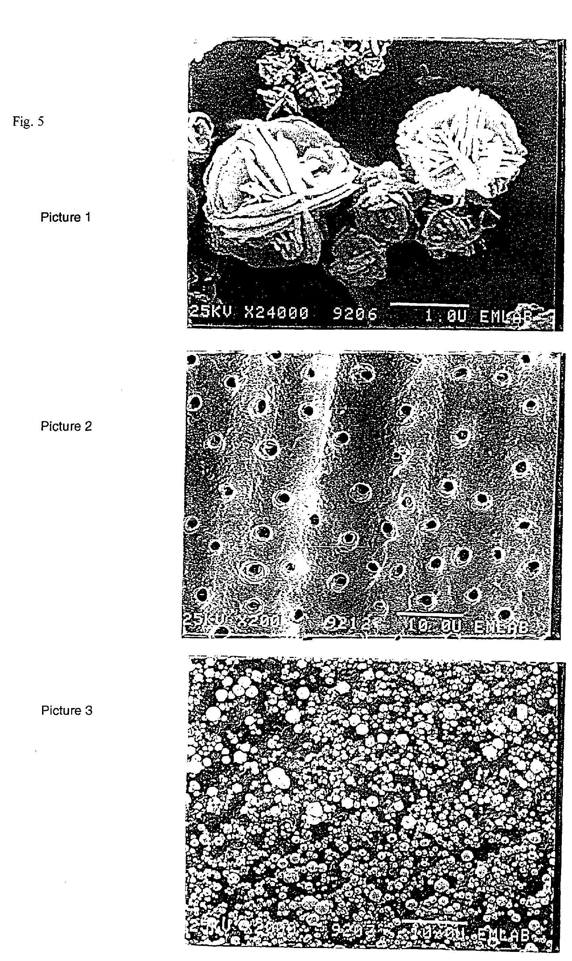

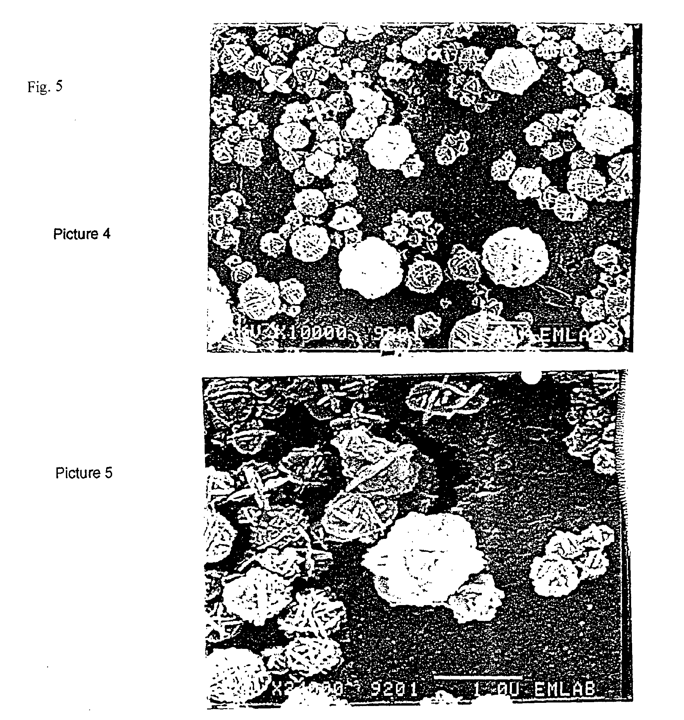

The tooth surfaces used came from extracted teeth of which the roots were removed 1 mm below the cementoenamel junction. Pulpal soft tissue was removed from the coronal pulp chamber A second section parallel to the first was made to remove the occlusal enamel and create a flat occlusal plane of dentin. The resulting crown segment was glued to a 2x2xO.6 cm piece of Plexiglas penetrated by a needle.
Apart from conductance experiments the tooth surfaces were challenged for 60' with the new mouth washes.Scanning electron microscopy was done on samples to determine if any crystalline deposits were created on the dentin surface. Half of them were rinsed with water for 10 sec and allowed to air dry overnight. The other half were stored in water at 37'C for 24 hrs, rinsed for 10 sec with water and air-dried overnight before being sputter coated and examined in a JEOL SEM. The water soaking step was used to determine if treated smear layers might dissolve slowly and to evaluate the solubility of any crystalline deposits that developed on dentin over the 60 min treatment time.
The scanning electron microscopy of acid-etched dentin treated with the mouthwashes for 60 min revealed a significant amount of crystalline precipitate on dentin When these specimens were examined at 24,000x, the crystal aggregates were seen to be ball-like clumps of plate and needle-like crystals (0.05 x 0.3 microns) which were sometimes oriented to form an "x" on the balls (Figure 5, Picture 1).

Scanning electron microscopy of acid-etched dentin treated with the comparators for 60 min showed no evidence of crystalline deposits (Figure 5, Picture 2). When smear layer-covered dentin was treated with the mouthwashes for 60 min, a large number of crystalline deposits could be seen at low power (Figure 5, Picture 3) When viewed at a higher power, one could see a wide variety of crystal types on top of the smear layer (Figure 5, Picture 4) indicating that the product had little effect on the smear layer. Higher magnification (Figure 5, Picture 5) revealed ball-like aggregates composed of needle-like crystals often oriented to form an X.
When smear layer-covered dentin was treated with the comparator for 60 min, most of the tubules remained occluded with smear plugs, although some superficial smear layer was lost as evidenced by the presence of cracks over each tubule. No crystal deposits were seen

### Conclusion

The results clearly show the superiority of the new product over existing products to deposit strontium on the tooth surface

### 3. PRECIPITATION DATA

Addition of saliva to solutions of Sr²⁺ or Ca²⁺ and sodium mono fluorophosphates (MFP) caused an enhanced formation of precipitate from the solutions if certain concentrations of the ingredients were exceeded; Whereas a minor amount of precipitation forms with strontium chloride hexahydrate or calcium chloride dihydrate at concentrations of 5 % or higher in the presence of 0.5% MFP there was a major increase in precipitation at 8 times lower levels of the cations and MFP when saliva was added.

Similar, slowly forming precipitates can also be obtained from 2 phases using calcium ions in one phase and carbonate (CO₃²⁻) in the other. This might also be applied to the problem of occluding dentinal tubules.

### 4. ACID TEST

The permeability of dentinal tubules was measured after treatment with acidic solutions ( pH 4) in the presence or absence of free fluoride and mixed with strontium ions.
The solutions which did not have any free fluoride contained MFP . An isotonic saline solution was used as a comparator.
The improvement was defined as the ratio of the transmission increase in the presence of free fluoride over the increase in the absence of free fluoride

Effect of experimental desensitizing solutions on the permeability

| Of dentin, *in vitro.* | | | | | |
|---|---|---|---|---|---|
| Treatment solution | | 1' treat ment | 10' treat ment | 60' treat ment | 24 h soak |
| A+A (1) | SrCl2 -NaF (free fluoride; pH 4) | 110 | 112 | 115 | 179 |
| B+B (2) | SrCl2-MFP(bound fluoride;pH 4) | 114 | 141 | 170 | 267 |
| Placebo (3) | NaCI (pH 7) | 102 | 96 | 90 | 96 |
| Improvement | (2)-(1) | 33% | 64% | 69% | 52% |
| | (2)-(3) | | | | |

| Effect of experimental desensitizing solutions on the permeability of smear layer - covered dentin, *in vitro.* | | | | |
|---|---|---|---|---|
| Treatment solution | 1' | 10' | 60' | 24 h soak |
| (1) | 106 | 104 | 103 | 198 |
| (2) | 156 | 184 | 160 | 271 |
| (3) | 112 | 94 | 99 | 115 |
| Improvement | 100% | 89% | 93% | 47% |

### 5. CLINICAL DATA

### Protocol

The trial was a double blind study carried out by three investigators, there being one group of subjects receiving the active mouthwash and another a placebo control. Informed consent to participate was obtained from forty patients with dentine hypersensitivity. Patients were over 18 years old, in good general health, suffered from no active dental disease, and had at least two adjacent teeth which were sensitive to stimulation both with an air jet and mechanical stimulation with a probe. They were randomly allocated by an independent agency to the active and control groups.

Upon recruitment, patients were given routine prophylaxis and checked for caries, erosion or abrasion. Two weeks after completion of any dental work each patient was issued with a supply of fluoride-free toothpaste and suitable multifilament toothbrushes and was instructed to use these alone until completion of the trial. One week after issue of the paste and brushes sensitivity was assessed in two non-adjacent teeth.This assessment was used as a baseline for each patient to which changes was determined during the trial period were related.

Sensitivity was subjectively assessed by each patient using a Visual Analogue Scale as described by Scott & Huskisson (1976). A 10 cm long ungraduated horizontal line was used with the extremities labelled "No discomfort" and "Severe discomfort" respectively. Patients were instructed to place a cross on the line corresponding to where their sensitivity following stimulation lay. An air jet stimulus was applied to the cervical margin of each selected tooth followed five minutes later by mechanical stimulation with prompting from the operator or referral to any previous recording.

Mouthwashing was carried out for six weeks. The active formulation was presented in vials. The placebo was presented in vials identical to those containing the active formulation but both vials contained 5 ml of an aqueous solution of sodium chloride (0.5 % w/v). Instructions for the use of both systems were identical, namely that the two solutions be mixed and the mixture then used immediately as a mouthwash for one minute then expectorated. This was followed by a water rinse and then by toothbrushing with the fluoride-free paste. The complete procedure was carried out twice daily throughout the treatment period. Sensitivity assessments were made fortnightly during the mouthwashing period and six weeks after cessation of the treatment. Following collection of the data a graduated line divided into 10 equal subdivisions was superimposed on each visual analogue scale and a reading of 1-10 recorded. Active and control patients were then identified and separated into .two groups. Analysis was carried out by individual rather than by tooth, using the probe-stimulated and air-stimulated mean recordings for the two teeth, providing a single pain score for each stimulus or each occasion in each patient Median pain scores were calculated for each group/occasion/stimulus. Differences between groups wer assessed by the Mann-Whitney test and change of pain response with time within individuals for each group/stimulus by the Wilcoxon matched-pair,signed rank test.

### Results and Discussion

Of the forty patients who gave informed consent thirty-five completed the trial, four failed to attend at the required intervals and one was excluded having developed Patient acceptance of both regimes was good and many patients were enthusiastic about the perceived benefits.

The results of the individual pain scores using the air stimulation after 2, 4 or 6 weeks of treatment and 6 week after cessation of the treatment are shown in the following figures.

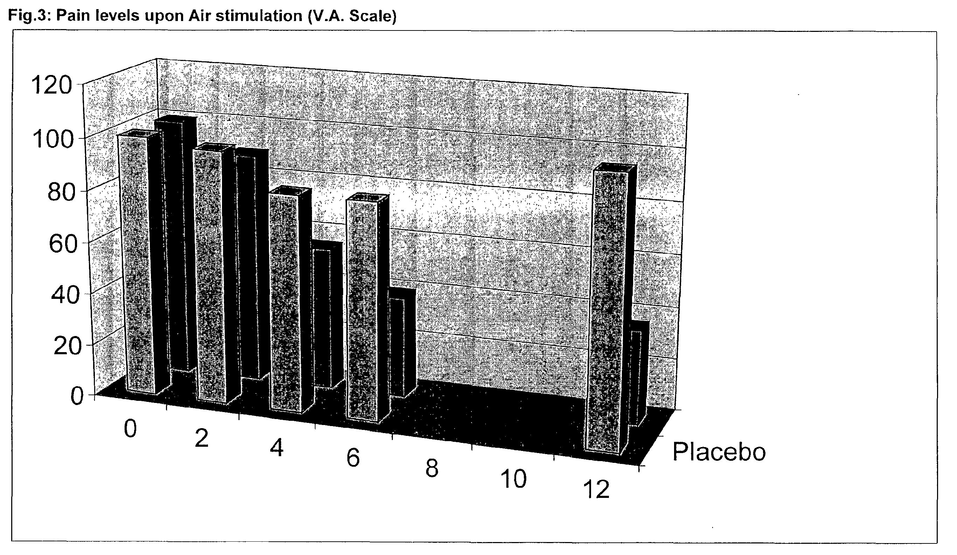

Fig. 3 summarizes the pain levels upon the air stimulus for the placebo group and the active group and compares them with the baseline pain levels.

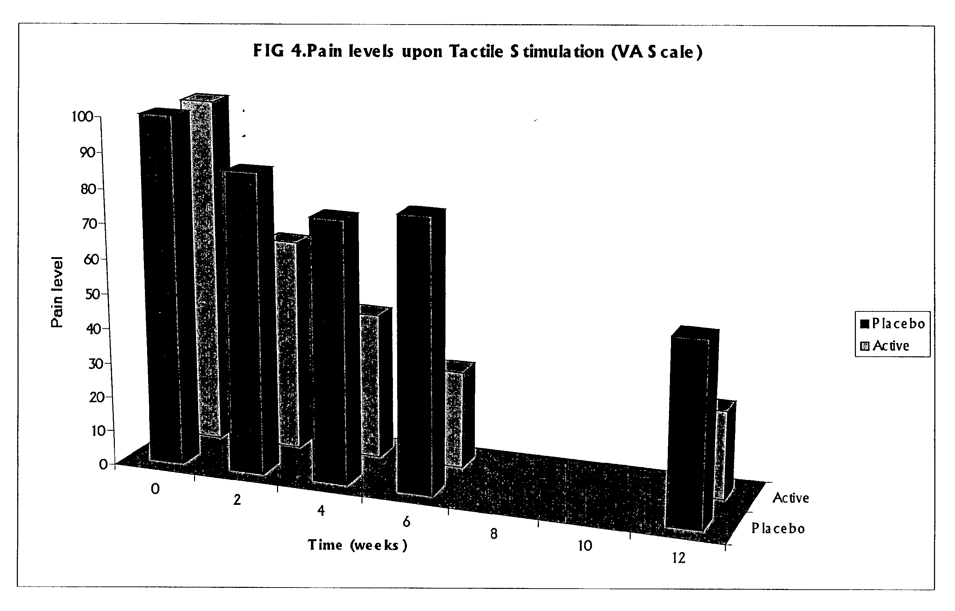

Fig. 4 summarizes the pain levels - using the tactile stimulus for the placebo group
It is evident that the placebo effect is much less pronounced with the air stimulation (Fig. 3) and therefore the improvement in pain with the active ingredient can clearly be seen.

### Conclusion

This study was designed to assess the efficacy of a mouthrinse in the treatment of dentine hypersensitivity. the protocol was such that the effects of other variables , particularly the toothpaste used by the individual were minimised . Thus the same fluoride-free toothpaste and type of brush to be used alongside the mouthwashes were provided for each patient. Such a study is essential at this stage of development since it should-in our present state of knowledge of dentine hypersensitivity-indicate whether there is a beneficial effect of the active preparation.
The results have clearly shown that the proposed product is superior in its desensitizing properties to any of the presently commercially-available products.

## Claims

1. A system for preparing a desensitizing composition comprising strontium and fluoride, said system comprising a source of first and second compositions said compositions being segregated and containing respectively a physiologically acceptable source of free strontium ions and a physiologically acceptable source of free fluoride ions and dispensing means arranged to dispense said compositions so that a mixture thereof ,immediately prior to application thereof to the teeth, causes a fine precipitate to form which partially -or completely-blocks the dentinal tubules.

2. A system for preparing a desensitizing composition comprising calcium and carbonate, said system comprising a source of first and second compositions said compositions being segregated and containing respectively a physiologically acceptable source of free calcium ions and a physiologically acceptable source of free carbonate ions and dispensing means arranged to dispense said compositions so that a mixture thereof ,immediately prior to application thereof to the teeth, causes a fine precipitate to form which partially -or completely-blocks the dentinal tubules.

3. A system according to Claim 1 or Claim 2 wherein said segregated compositions are housed in a common container and are separated from one another by a barrier which prevents mixing prior to the compositions being dispensed.

4. A system according to any preceding claim wherein the source of strontium ion is strontium chloride.

5. A system according to any preceding claim wherein the source of calcium ion is calcium chloride.

6. A system according to any preceding claim wherein the source of fluoride ion is acidulated phosphate fluoride.

7. A system according to any preceding claim wherein the source of fluoride ion is sodium fluoride.

8. A system according to any preceding claim wherein the source of carbonate ion is sodium carbonate.

9. A system according to any preceding claim wherein each of said segregated compositions is in the form of a paste or gel.

10. A system according to any one of Claims 1 to 7 wherein each of the said segregated compositions is in the form of a liquid solution.

11. A system according to any preceding claim wherein said strontium compound is present in a concentration of between 0.1 and 12%.

12. A system according to any preceding claim wherein said calcium compound is present in a concentration of between 0.1 and 12%.

13. A system according to any preceding claim wherein the concentration of fluoride compound is between 0.001% and 4%.

14. A system according to any preceding claim wherein the concentration of carbonate compound is between 0.001% and 4%.

15. A kit for desensitizing the dental area of a patient having hypersensitive teeth which comprises supplies of two separate dentifrice or mouthwash compositions, one of said compositions containing a source of a physiologically acceptable free strontium or calcium ion and the other of said compositions containing a physiologically acceptable source of free fluoride or carbonate ion, and optionally instructions for admixing said phases on or immediately prior to application to the teeth of the patient to form on the surface of the teeth strontium and fluoride- or calcium and carbonate- in a form to effect desensitization of the dental area of the patient.

16. The use of a physiologically acceptable source of fluoride to produce industrially a kit or system according to claims 1 to 14 for the treatment of teeth in a combination therapy.

17. The use of a physiologically acceptable source of carbonate to produce industrially a kit or system according to claims 1 to 14 for the treatment of teeth in a combination therapy.

18. The use of a physiologically acceptable source of strontium to produce industrially a kit or system according to claims 1 to 14 for the treatment of teeth in a combination therapy.

19. The use of a physiologically acceptable source of calcium to produce industrially a kit or system according to claims 1 to 14 for the treatment of teeth in a combination therapy.
